**Europäisches Patentamt**

**European Patent Office**

**Office européen des brevets**

(19)

(11) Publication number: **0 122 814**

**A2**

(12) # EUROPEAN PATENT APPLICATION

(21) Application number: **84302649.3**

(22) Date of filing: **18.04.84**

(51) Int. Cl.³: **C 12 N 15/00**
**C 12 N 5/00, C 12 P 21/00**
**G 01 N 33/54, A 61 K 39/395**

(30) Priority: **18.04.83 GB 8310403**

(43) Date of publication of application:
**24.10.84 Bulletin 84/43**

(84) Designated Contracting States:
**CH DE FR GB LI SE**

(71) Applicant: **Mach, Bernard François**
**45, Route de Pregny**
**CH-1292 Chambesy/GE(CH)**

(71) Applicant: **Raboudin-Combe, Chantal**
**Residence Bellevue Route de Thonon**
**F-74140 Douvaine(FR)**

(71) Applicant: **Gorski, Jacek Andrew**
**114 rue des Eaux Vives**
**Genève(CH)**

(72) Inventor: **Mach, Bernard François**
**45, Route de Pregny**
**CH-1292 Chambesy/GE(CH)**

(72) Inventor: **Raboudin-Combe, Chantal**
**Residence Bellevue Route de Thonon**
**F-74140 Douvaine(FR)**

(72) Inventor: **Gorski, Jacek Andrew**
**114 rue des Eaux Vives**
**Genève(CH)**

(74) Representative: **Bannerman, David Gardner et al,**
**Withers & Rogers 4 Dyer's Buildings Holborn**
**London, EC1N 2JT(GB)**

(54) A method of cotransformation, cotransformed mouse cells producing at their surface human class II antigens and the use of those cells to generate mouse hybridomas that produce monoclonal antibodies to those antigens.

(57) A method of contransforming appropriate host cells with at least two distinct DNA sequences whose products ineract with each other after production in the host cell. Mouse cell lines engineered by that method to produce at their surface human Class II antigens of the major histocompatibility complex. The mouse cell lines of this invention are cotransformed with at least a DNA sequence encoding the α-chain of the selected human Class II antigen, a DNA sequence encoding the β-chain of that antigen and a DNA sequence encoding a selectable marker. More preferably, the mouse cells are also cotransformed with a DNA sequence encoding the In chain of that antigen. These mouse cells are useful in producing those antigens and in forming hybridomas that are characterized by monoclonal antibodies recognizing allospecific determinants of those antigens. These antibodies are useful in typing and therapeutic processes and compositions.

EP 0 122 814 A2

Croydon Printing Company Ltd

A METHOD OF COTRANSFORMATION, COTRANSFORMED
MOUSE CELLS PRODUCING AT THEIR SURFACE HUMAN
CLASS II ANTIGENS AND THE USE OF THOSE CELLS
TO GENERATE MOUSE HYBRIDOMAS THAT PRODUCE
MONOCLONAL ANTIBODIES TO THOSE ANTIGENS

## TECHNICAL FIELD OF THE INVENTION

This invention relates to a method of cotransformation and mouse cells lines engineered by that method
to produce at their surface human Class II antigens of
the Major Histocompatibility Complex. More specifically,
it relates to processes used to produce cotransformed mouse
cells that produce at their surface human Class II antigens
and the cotransformed mouse cells themselves. These mouse
cells are useful in producing hybridomas that make monoclonal antibodies that recognize allospecific Class II
determinants. Such specific monoclonal antibodies are
employed in typing individuals for tissue transplantation
and for determining their susceptibility to certain diseases and also as therapeutic agents.

## BACKGROUND ART

The human lymphocyte antigen ("HLA") system is
the Major Histocompatibility Complex in man. It, therefore, constitutes the strongest barrier for tissue and
organ transplants between individuals, apparently distinguishing between self and non-self. In addition, HLA
factors have been demonstrated to be associated with increased susceptibility to a wide variety of diseases.
Therefore, the antigens of the HLA system and their antibodies have found use in diagnostic typing processes and products for determining an individual's susceptibility to a wide variety of diseases and his characteristics as a donor or acceptor of a tissue or organ transplant [F. H. Bach and J. J. Van Rood, N. Engl. J. Med., 295, pp. 806-13 (1976)].

From a genetic point of view the HLA system is fairly well characterized. See e.g., L. P. Ryder et al., "Genetics Of HLA Disease Association", Ann. Rev. Genet., 15, pp. 169-87 (1981); J. L. Strominger et al., in The Role of the Major Histocompatibility Complex in Immunobiology, M.Dorf, ed., Garland SPTM·Press, pp. 115-172 (1981); T. Sasazuki et al., "The Association Between Genes In The Major Histocompatibility Complex And Disease Susceptibility", Ann. Rev. Med., 28, pp. 425-52 (1977). See also "Structure And Function Of HLA-DR", Immunological Reviews, 66 (1982). It consists of a series of more or less highly polymorphic loci situated within an interval of about 2 centimorgan (cM) on the short arm of chromosome 6. Three loci in that system (HLA-A, B and C) encode one class of codominantly expressed alloantigens (Class I). Another locus (HLA-D) encodes a second class of codomi-nant alloantigens with a high degree of recognized polymorphism (Class II). Three other loci, controlling some of the initial components (C2, C4 and factor Bf) of the complement cascade, also belong to the HLA system (Class III). Finally, there is an non-specific region in the HLA complex designated Ia. Region Ia appears related to, but different than, the DR locus.

The biology of the HLA system is less well understood. Class I antigens are distributed in all tissues except erythrocytes. Class II antigens are highly polymorphic and are substantially restricted to $\beta$-lymphocytes and mononuclear phagocytic cells. The Class III complement factors are directly involved in the activation of the C3 factor, the key component in the complement system. The Class II antigens appear to be involved in immunological phenomena -- immune responsiveness, T-cell suppression, T-cell and β-cell cooperation and T-cell and macrophage presentation [B. Benacerraf in "The Role Of The Major Histocompatibility Complex In Immunobiology", M. E. Dorf, ed., Garland SPTM Press, pp. 255-69 (1981)].

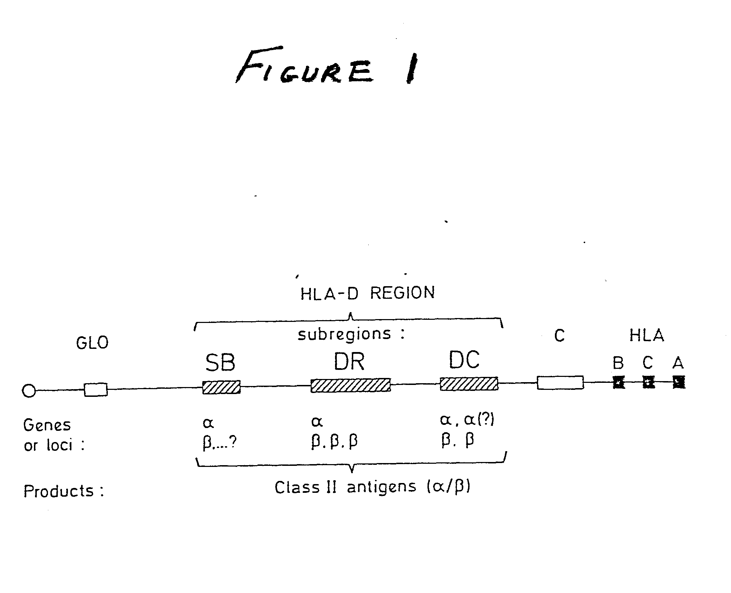

The human Class II antigens are composed of two transmembrane, non-covalently-linked glycosylated peptide chains, a heavy or α-chain of about 35000 molecular weight and a light or β-chain of about 29000 molecular weight, that span the cellular membrane [Strominger et al., supra; and Ryder et al., supra]. Intracellularly, a third peptide chain, the invariant chain (In), of about 32000 molecular weight is associated with the α- and β-chains [D. J. Charron and H. O. McDevitt, J. Exp. Med., 152, pp. 185-365 (1980); Strominger, supra]. It appears that the light or β-chain carries the polymorphism of the Class II antigens, while the α-chain and third chain appear identical in different individuals [G. Corte et al., Proc. Natl. Acad. Sci. USA, 78, pp. 534-38 (1981); Charron and McDevitt, supra]. It is believed that the In chain is involved in the assembly and transport of the Ia or Class II antigens [M.J. Owen et al., J. Biol. Chem., 256, pp. 8987-93 (1981); S. Kuist et al., Cell, 29, pp. 61-69 (1982)]. At least three distinct types of Class II antigens have been identified -- HLA-DR antigens F.H. Bach and J.J. van Rood, N. Engl. J. Med., 295, pp. 806-13 (1976); W.H. Bodmer et al., in Histocompatibility Testing (eds. W. Bodman et al.), Munksgaard, Copenhagen, Denmark (1978); R.J. Winchester and H.E. Kunkel, Adv. Immunol., 28, pp. 221-298 (1980); J.L. Strominger et al., in The Role Of The Major Histocompatibility Complex In Immunobiology, (ed. M.E. Dorf), Garland, New York, New York, (1981), the DC antigens (R. Tosi et al.), Exp. Med., 148, pp. 1592-1611 (1978), and the SB antigens (S. Shaw et al., J. Exp. Med., 152, pp. 565-80 (1980)). See Figure 1. These antigens are characterized by serological and cellular alloreactivity, with the HLA-DR antigens displaying the most extensive polymorphism.

Among the HLA-DR antigens a number of serologically distinct HLA-DR antigens have been identified -- HLA-DR1 through HLA-DR8 -- and monoclonal antibodies have defined subparts of DR antigens within homozygous cell lines [V. Quaranta et al., J. Immunol., 125, pp. 1421-25 (1980); S. Carrel et al., Mol. Immunol., 18, pp. 403-11 (1981)]. Two DR β-chains can also be distinguished in several homozygous cell lines by peptide analysis [R. S. Accolla et al., Proc. Natl. Acad. Sci. USA, 78, pp. 4549-51 (1981)]. Several other loci also exist that encode polymorphic Ia-like antigens that are closely linked but not identical, to HLA-DR [G. Corte et al., Nature, 292, pp. 357-60 (1981); Nadler et al., Nature, 290, pp. 591-93 (1981)]. We have also established that at least three different loci exist for HLA-DR or DR-like β chains [J Gorski et al., in press; E.O. Long et al., Hum. Immunol., in press]. Therefore the HLA-DR subregion of the HLA-D region is itself genetically complex.

At present HLA-DR antigens are isolated serologically by precipitation with antisera. Therefore, the exact nature of the HLA-DR determinants is uncertain. However, these antigens have found use in typing processes and products to determine the compatibility of donors and acceptors for tissue or organ transplants and to determine susceptibility of an individual to a wide variety of diseases. For example, Ryder et al., supra, has reported the following disease susceptibilities based on DR1 through DR8 typing:

| Disease | Typing | Positive Frequency (%) Patients | Controls | Relative Risk | Ethiological Fraction Δ |
|---|---|---|---|---|---|
| Dermatitis herpetiformis | D/DR3 | 85 | 26.3 | 15.4 | 0.80 |
| Coeliac disease | D/DR3 | 79 | 26.3 | 10.8 | 0.72 |
| | D/DR7 also increased | | | | |
| Sicca syndrome | D/DR3 | 78 | 26.3 | 9.7 | 0.70 |
| Idiopathic Addison's disease | D/DR3 | 69 | 26.3 | 6.3 | 0.58 |
| Graves' disease | D/DR3 | 56 | 26.3 | 3.7 | 0.42 |
| Insulin-dependent diabetes | D/DR3 | 56 | 28.2 | 3.3 | 0.39 |
| | D/DR4 | 75 | 32.2 | 6.4 | 0.63 |
| | D/DR2 | 10 | 30.5 | 0.2 | -- |
| Myasthenia gravis | D/DR3 | 50 | 28.2 | 2.5 | 0.30 |
| | B8 | 47 | 24.6 | 2.7 | 0.30 |
| SLE | D/DR3 | 70 | 28.2 | 5.8 | 0.58 |
| Idiopathic membraneous nephropathy | D/DR3 | 75 | 20.0 | 12.0 | 0.69 |
| Multiple sclerosis | D/DR2 | 59 | 25.8 | 4.1 | 0.45 |
| Optic neuritis | D/DR2 | 46 | 25.8 | 2.4 | 0.27 |
| Goodpasture's syndrome | D/DR2 | 88 | 32.0 | 15.9 | 0.82 |
| Rheumatoid arthritis | D/DR4 | 50 | 19.4 | 4.2 | 0.38 |
| Pemphigus | D/DR4 | 87 | 32.1 | 14.4 | 0.81 |
| IgA nephropathy | D/DR4 | 49 | 19.5 | 4.0 | 0.37 |
| Hydralazine-induced SLE | D/DR4 | 73 | 32.7 | 5.6 | 0.60 |
| Hashimoto's thryoiditis | D/DR5 | 19 | 6.9 | 3.2 | 0.13 |
| Pernicious anemia | D/DR5 | 25 | 5.8 | 5.4 | 0.20 |
| Juvenile rheumatoid arthritis: pauciart | D/DR5 | 50 | 16.2 | 5.2 | 0.40 |

From these typings, it can be seen that an individual typed positive for D/DR4 has a 6.4 times higher risk of developing insulin-dependent diabetes than individuals typed negative for D/DR4. See also "HLA-DR And Diseases", Immunological Reviews, 70 (1983); J. D. Stobo et al., J.Lab.Clin.Med., 100, pp. 822-28 (1982); J. T. Rosenbaum et al., J.Exp.Med., 15, pp. 1694 et seq. (1981); P. Steinmark et al., Proc.Natl. Acad.Sci.USA, 78, pp. 7111 et seq. (1981).

In some cases it has also been demonstrated that a disease is more severe in patients having the disease-associated antigen than in those who do not have that antigen. For example, multiple sclerosis progresses more rapidly in D/DR2-positive patients than in D/DR2-negative patients. Moreover, relapses in certain diseases are more common in patients positive for the disease-associated antigens.

Plainly, then, HLA-DR typing has great diagnostic and prognostic value.

However, the use of such typing processes and products and, therefore, the attainment of the important advantages that they would provide in identifying acceptable transplant donors and recipients and disease-susceptible individuals, has been severely restricted because the present typing procedure is not able to distinguish well among various class II specificities because the monoclonal antibodies available to date are not specific and distinct enough. Moreover antibodies against each individual HLA-DR, DC and SB antigen are not available.

## DISCLOSURE OF THE INVENTION

This invention generally solves the problems referred to above by providing a process of cotransformation and the use of that process to cotransform mouse cells so that those cells express at their surface particular human class II antigens. The invention also provides these mouse cells carrying at their surface human class II antigens. Because this surface antigen is the only non-mouse antigen produced by the mouse cell, it will elicit a specific immune response in mice of the same genetic type and thereby allow the preparation hybridomas that produce monoclonal antibodies specific to the particular human class II antigen produced by the mouse cell.

By virtue of our invention, it is now possible to prepare mouse cells that express a particular human class II antigen in a stable manner at the cell surface. Moreover, as a result of this invention, mouse-mouse hybridomas may be formed that produce monoclonal antibodies recognizing allospecific class II determinants expressed by the mouse cells of this invention.

Finally, this invention provides such allospecific monoclonal antibodies for use in typing individuals for tissue transplants and for their susceptibility to certain diseases and for use as therapeutic agents against various diseases in which a class II antigen-linked immune reactivity may be involved in the pathological process.

These and the other objects of this invention are effected by a general method of cotransformation where the products of the DNA sequences used in the cotransformation interact subsequently to their production. In accordance with this invention, this method in one preferred embodiment results in mouse cells cotransformed with DNA sequences encoding the α, β, and perhaps the invariant chain, of human class II antigens that produce the desired human class II antigens on their surface. Such mouse cells may then be employed to produce mouse-mouse hybridomas that produce monoclonal antibodies recognizing allospecific class II determinants.

The contransformation process of this invention comprises the steps of cotransforming appropriate cells with at least two DNA sequences, said sequences encoding protein products that interact with each other after synthesis in the host cell.

In one preferred embodiment of this invention, that process is employed to produce mouse cells carrying at their surface a human class II antigen.

The process for producing those mouse cells comprises the step of cotransforming appropriate mouse cells with (a) a DNA sequence encoding the α-chain of a human class II antigen, (b) a DNA sequence encoding the β-chain of that human class II antigen, (c) a DNA sequence encoding a selectable marker, and preferably, but not necessarily, (d) a DNA sequence encoding the invariant chain of that human class II antigen. These cells may then be cultured to express those DNA sequences, to assemble intracellularly the protein products of them correctly and to transmit the resulting protein complex to the cell surface with the correct antigenic specificity. The resulting mouse cells carrying that antigen at their surface may then be employed to produce hybridomas that produce monoclonal antibodies recognizing allospecific determinants of the class II antigen for use in typing processes and therapy.

## BRIEF DESCRIPTION OF THE DRAWINGS

Figure 1 is a schematic outline of the major histocompatibility complex and the various regions therein.

Figure 2 is a partial restriction map of DNA sequence λ DR β-10 used in one embodiment of this invention.

## BEST MODE OF CARRYING OUT THE INVENTION

In order that the invention described herein may be more fully understood, the following detailed description is set forth.

In the description the following terms are employed:

Recombinant DNA Molecule -- A molecule consisting of segments of DNA from different genomes.

Polypeptide -- A linear array of amino acids connected one to the other by peptide bonds between the α-amino and carboxy groups of adjacent amino acids.

Expression -- The process undergone by a DNA fragment or gene to produce a polypeptide. It is a combination of transcription (the process of producing mRNA from a DNA fragment) and translation (the process of producing a polypeptide from mRNA).

Plasmid -- A non-chromosomal double-stranded DNA sequence comprising an intact "replicon" such that the plasmid is replicated in a host cell. When the plasmid is placed within a unicellular organism, the characteristics of that DNA of the plasmid modifies or transforms the characteristics of the host organism. For example, a plasmid carrying the gene for tetracycline resistance ($Tet^R$) transforms a cell previously sensitive to tetracycline into one which is resistant to it. A cell transformed by a plasmid is called a "transformant".

Cloning -- The process of obtaining a population of organisms or DNA sequences derived from one such organism or sequence by asexual reproduction.

Cotransformation -- A process of transfecting appropriate cells with at least two distinct DNA sequences encoding proteins that interact after production in the host cell with each other. The cells that result from
this process are called a "cotransformants".

As described above, the cotransformation process
of this invention comprises the step of cotransforming an
appropriate host cell with at least two DNA sequence that
encode distinct protein products, the protein products of
the sequences interacting with each other after their
production in the host cell.

In the general process of this invention, a wide
variety of DNA sequences and host cells may be employed.
For example, the host cells useful in the process may be
eukaryotic or prokaryotic hosts, such as <u>E.coli</u>, <u>Bacillus</u>,
yeast and plant and animal cells. Preferably, eukaryotic
cells are used and most preferably mouse cells.

The DNA sequences useful in this invention may
also vary widely. For example, a DNA sequence coding for
a particular protein and DNA sequences encoding the one
or more enzymes that are required to glycosylate that protein may be used. In the preferred embodiment of this
invention, which is described in more detail below, we
employ the DNA sequences encoding the α and β-chains of
human class II antigens, and preferably also the DNA
sequence encoding the invariant (In) chain in our
cotransformation.

As described above the mouse cells of this invention are characterized by their cotransformation with DNA
sequences encoding the α, β chains and preferably, but
not necessarily DNA sequences encoding the invariant chains
(In) of human class II antigens and which as a result of
that cotransformation express those DNA sequences, assemble
their protein products intracellularly and transmit the
assembled complex to the cell surface with the correct
antigenic specificity. Such cotransformed cells may then
be employed to produce hybridomas and from them monoclonal
antibodies recognizing allospecific determinants of those
class II antigens.

There are at least three distinct types of class II
antigens and antibodies that may be produced in accordance with this invention. Each is encoded in the HLA-D region (Figure I). They include the HLA-DR antigens [F. H. Bach & J. J. VanRoad, N. Engl. J. Med., 295, pp. 806-13 (1976); W. H. Bodmer et al., in Histocompatibility Testing, eds. W. H. Bodmer et al., Munksgaard, Copenhagen, Denmark (1978); R. J. Winchester and H. G. Kunkel, Ads. Immunol., 28, pp. 221-98 (1980); J. L. Stromiger et al., in The Role Of The Major Histocom-patibility Complex In Immunology, ed. M. E. Dorf, Garland, New York, pp. 115-72 (1981)]; the HLA-DC antigens [R. Tosi et al., J. Exp. Med., 148, pp. 1592-1611 (1978)] and the HLA-SB antigens [S. Shaw et al., J. Exp. Med., 152, pp. 565-580 (1980)]. Moreover, this invention is also applicable to other species of human class II antigens.

DNA sequences encoding these various class II antigens are available. For example, cDNA clones for the polymeric β chain of HLA-DR and HLA-DC are described in E. O. Long et al., Proc. Natl. Acad. Sci USA, 79, pp. 7465-69 (1982); cDNA clones for the non-polymeric DR α-chain are described in C. T. Wake et al., Proc. Natl. Acad. Sci. USA, 79, pp. 6969-83 (1982); cDNA clones for the HLA-DR α chain and HLA-DC α chains are described in J. S. Lee et al., Proc. Natl. Acad. Sci USA, 79, pp. 545-49 (1982) and C. Auffray et al., Proc. Natl. Acad. Sci USA, 79, pp. 6337-41 (1982); and cDNA clones for the HLA-DC β chains are described in K. Wiman et al., Proc. Natl. Acad. Sci. USA, 79, pp. 1703-07 (1982). In addition, cDNA clones coding for the DR-associated invariant chain (In) are also available. Moreover, these cDNA clones may be employed to select the genomic DNA sequences encoding the desired chains [J. Gorski et al., in press]. See also description and deposits infra. We prefer to use genomic clones in the process of this invention.

The mouse cells that are useful in accordance with this invention include generally all available mouse cell lines. These include, among others, mouse L cells [E. C. Lai et al., Proc Natl. Acad. Sci. USA, 77, pp. 244-48 (1980)], macrophages, and β-lymphocytes. We prefer to employ mouse L(tk⁻) cells in the preferred embodiments of this invention. It is surprising that such mouse cells express the human class II DNA sequences which we employ to cotransform them because mouse fibroblasts do not normally express their own class II genes. Accordingly, the human DNA sequences that we have cotransfected into the mouse cells by the processes of this invention are believed to be under a different expression control than the counterpart mouse genes.

Various methods of contransformation may be used in accordance with this invention. They may be selected from conventional transformation methods with due regard to the DNA sequences to be used and the characteristics of the host cells to be transformed. We prefer to employ DNA-mediated gene transfer into our preferred mouse cells substantially as described in A. Pellicer et al., Science, 209, pp. 1414-21 (1980) and E. C. Lai et al., Proc. Natl. Acad. Sci. USA, 77, pp. 244-48 (1980). For this cotransformation we employed at least three different DNA sequences: (a) a DNA sequence encoding the polymeric β chain of the desired Class II antigen; (b) a DNA sequence encoding the α-chain of that desired Class II antigen; and (c) a DNA sequence encoding a selection marker. It should, however, be understood that the DNA sequence encoding the invariant chain (In) of the desired Class II antigen is also preferably used in our cotransformations. However, this last DNA sequence may not be required, because some of our experiments demonstrate that the presence of the human invariant gene product in the cotransformed mouse cell is not essential for the surface production of the desired Class II antigen.

As selection markers, we prefer to employ the gene coding for Herpes Simplex Virus thymidine Kinase (tk) [M. Perucho et al., Cell, 22, pp. 309-17 (1980)]. However, it should be understood that a variety of other selection markers are also useful. These include, for example, antibiotic resistance markers, like resistance to G418, metabolic inhibition markers, like mycophenolic acid, and mutant markers, like HGPRT negative strains, grown in HAT medium. All that is required is that some means be available to exert a positive pressure on the selection of correctly cotransformed cells.

In the specific cotransformations described herein we linearized the genomic clones containing the various DNA sequences with restriction endonucleases that cut the clones outside of those DNA sequences. However, we did not ligate the various sequences together before cotransformation. It should, however, be understood that some or all of the DNA sequences employed in our cotransformations may be ligated in vitro before cotransformation of the chosen cells with them.

The cotransformed mouse cells of our invention express these DNA sequences and assemble their protein products intracellularly and then transport that complex to the cell surface in correct antigen form. This expression is stable and we have observed it in some of our transformants for over four months. Moreover, the production of the desired antigens is not lost upon cultivation or passage of the cotransformants.

The human Class II antigen-positive mouse cells of our invention provide a new and unique source of human Class II antigens. More importantly, these mouse cells may be employed using standard immunization and selection techniques to prepare hybridomas that produce monoclonal antibodies recognizing allospecific determinants of those class II antigens. These specific antibodies may then be usefully employed in typing and therapeutic agents and processes.

The monoclonal antibodies of this invention may be produced from the Class II antigens of this invention in a variety of standard ways. Preferably, the cotransformed mouse cells of this invention are employed to produce mouse-mouse hybridomas that produce the desired monoclonal antibodies.

The hybridomas of this invention are preferably prepared by first immunizing mice of the same genetic background as those used for our cotransformation with the cotransformed cells of this invention. This choice insures that the only non-self protein made by the spleen cells of the immunized mouse is the desired Class II antigen. Of course, if a host cell other than mouse had been used in the original cotransformation, the host to be immunized should also be from that genetic type. The isolated spleen cells are then employed in a conventional manner to prepare a hybridoma by cell fusion.

One example of a method of immunizing mice with an antigen and then employing spleen cells of that mouse in the formation of a mouse-mouse hybridoma to produce an antibody specific to that antigen is described in European patent application 44441. In our hybridoma preparations, we prefer to employ C3H mice for immunization with the cotransformed cells of this invention when we have used L cells for cotransformation. We also prefer to employ a plasmacytoma mouse line which is defective in the production of its own immunoglobulin chain for subsequent hybridoma formation.

We then screen the antibodies produced by resulting collection of hybridomas with a panel of cells having known DR specificities in microtiter wells and select for specific and partially specific binding to each antigen. This process enables us to select a specific monoclonal antibody recognizing the allospecific determinants of each Class II antigen.

The antibodies of this invention will be more useful than previous Class II antibodies because the antibodies of this invention will be specific or "private" to a particular Class II specificity. In the terms of this invention, we describe that "private" specificity as an allospecificity to the Class II determinants.

The allospecific antibodies produced in accordance with this invention may be employed in typing and therapeutic processes and compositions. Such typing and therapeutic uses of Class II antibodies are known. The antibodies of this invention, because they are allospecific,

—14—

will allow more specific typing and more effective therapy than previous antibodies. It should also be understood that for typing or therapy, it is not necessary to employ the entire antibody of this invention. Instead, fragments of the antibody may be employed. Such fragments will be especially useful in therapeutic application because fragments of mouse made monoclonal antibodies are far less likely to be immunogenic in human use than the complete antibody.

In order that this invention may be better understood, the following examples are set forth. These examples are for purposes of illustration only and this invention should not be considered to be limited by any recitation used therein.

## EXAMPLES

### A. COTRANSFORMATION OF MOUSE L (tk⁻) CELLS

We maintained the thymidine kinase (tk)-deficient mouse L cell line (Ltk⁻) (a gift of P. Goldstein) in monolayer cultures in Dulbecco's Modified Eagel medium (DME), supplemented with heated 10% fetal calf serum; 1 mM sodium pyruvate; and 1% streptomycin and penicillin.

We then cotransfected $3 \times 10^6$ Ltk⁻ cells in $25 \text{ cm}^2$ tissue culture flasks with our DNA sequences substantially as described by M. Wigler et al., Cell, 16, pp. 777-85 (1979). In this process, we incubated the Ltk⁻ cells for 7 h at 37°C with calcium phosphate DNA precipitates and trypsin-dispersed them into 9 wells of a 24 well tissue culture microplate (Costar). After 24 h, we switched our cultures to hypoxactive-aminopterin-thymidine (HAT) selection medium and continued to grow them. We scored for positive (tk⁺) cotransformants after 3-4 weeks and selected positive cotransformants from each well and maintained them in $25 \text{ cm}^2$ tissue culture flasks. We number our positive cotransformants $1_1$ to $1_9$ and $22_1$ to $22_9$.

For the above-described cotransformations, we employed the following DNA sequences:

### Transformation 1

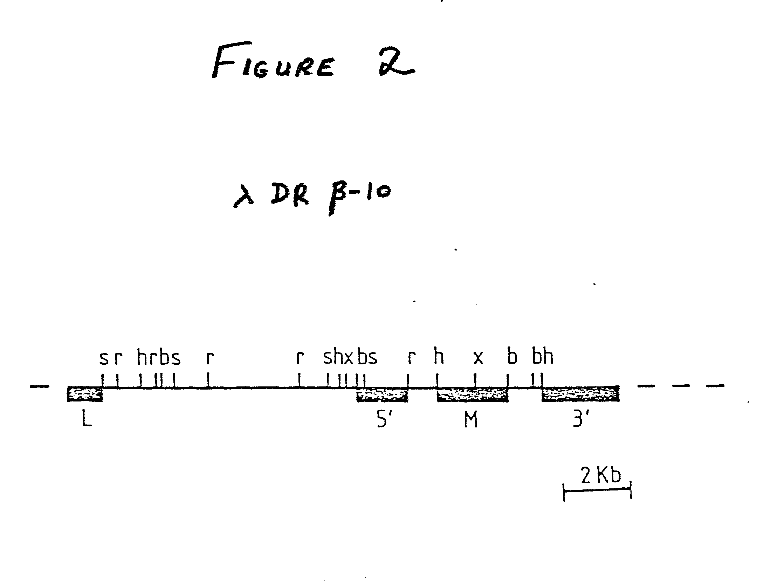

We prepared a DNA sequence encoding a HLA-DR β-chain by restricting genomic clone λ DR β-10 (Figure 2; see also infra), prepared from a DR 4, W 6 DNA library in lambda Charon 30 vector [J. Gorski et al., in press] with Nru and employed 1 µg of that linearized fragment per 19 µg of Ltk+ DNA carrier. We also prepared 50 ng of HindIII-linearized plasmid pAGO which carries the tk gene as a selective marker [F. Colbere-Garapin et al., Proc. Natl. Acad. Sci USA, 76, pp. 3755-59 (1979)]. The transformation efficiency of this transformation was 18 for the 9 wells.

### Cotransformation 22

In cotransformation 22, we employed 1 µg of the above described Nru linearized genomic clone λ DR β-10 and also employed 1 µg of an HLA-DR α chain-related genomic DNA sequence [A. J. Korman et al., Proc. Natl. Acad. Sci USA, 79, pp. 6013-17 (1982)] in λ DR α-HLA. Finally, we employed 3 µg of Sal I-linearized cos In [infra] which contained a DNA sequence encoding the human invariant chain (In) in cosmid vector pOPF1 [F. G. Grosveld et al., Gene, 13, pp. 227-37 (1980)]. This vector also provides the tk gene for use in selection. We employed these DNA sequences together with 14 µg of Ltk+ DNA carrier in cotransformation 22. The transformation efficiency in this cotransformation was 50 colonies for the 9 wells.

### B. DNA SEQUENCE EXPRESSION FROM COTRANSFORMED MOUSE L (tk⁻) CELLS

We first analyzed our $l_1$-$l_9$ transformants and $22_1$-$22_9$ cotransformants at the mRNA level using Northern blots. These analyses demonstrated that our selected tk+ cotransformants transcribed the DNA sequences encoding the human Class II antigens which we had used to transform them.

We than analyzed our tk+ cotransformed cells for the presence of cell-surface HLA-DR antigen using three HLA-DR monoclonal antibodies [R. S. Accolla et al.,
Eur. J. Immunol., 12, pp. 166-69 (1982); S. Carrel et al.,
Mol. Immunol., 18, pp. 403-11 (1981)]. We employed three
methods in this analysis: a cellular binding radioimmune
assay, immunofluorescence analysis and immunoprecipitation
of iodinated cell-surface proteins followed by polyacrylamide gel analysis. In each analysis, we included as controls Ltk⁻ cells, Ltk⁻ cells transformed with the DR β-chain
coding DNA sequence alone (e.g. our transformant $l_4$), a
human lymphoblastoid cell line HLA-DR W6, and control antibodies (such as HLA-A, B, C and anti H-2K$^K$).

For the cell binding assays we distributed
$5 \times 10^6$ cells in wells of V-bottom microplates (Dynatech
Corp.), centrifuged them at 4°C and resuspended them in
25 μl DME/2% FCS, containing $10^{-2}$ or $10^{-3}$ antibody dilu-
tion. After incubating the cells at 4°C with gentle shaking for 2 h, we washed the cells three times with
DME/2% FCS and resuspended them in 25 μl of that media,
containing approximately 3.5 ng ($10^5$ cpm) of $^{125}$I-protein A
(Amersham Int.) After four washes with DME/2% FCS, we
measured the cell-associated radioactivity in a gammma
counter.

In our cell binding assays we employed the following antibodies: (1) B9.12.1 anti HLA-A, B, C, monoclonal antibody (protein A-purified), which recognizes a
monomorphic determinant on human Class I heavy chains
[N. Rebai and B. Malissen, B. Ann. Immunol, in press];
(2) control mouse ascites fluid; (3) normal mouse serum;
(4) 11.4.1 anti H-2K$^K$ monoclonal antibody (protein A-purified), which recognizes murine class I antigens [V. T. Oi
et al., in Carr. Top. Microbiol., eds M. Melchers et al.,
81, pp. 115-25 (1978)]; and (5) a mixture of D1-12, D4-22
and BT 2.2 anti HLA-DR monoclonal antibodies (ascites fluid),
which recognizes common determinants of HLA-DR β chains
shared by all DR specificities [R. S. Accolla et al.,
Eur. J. Immunol., 12, pp. 166-69 (1982); S. Carrel et al.,
Mol. Immunoli, 18, pp. 403-11 (1980)]. Accordingly, these
latter HLA-DR antibodies are "public" not "private" monoclonal antibodies.  We centrifuged (10000 rpm) all antibody preparations for 15 min at 4°C prior to use.

We have displayed the results of the cellular
binding radioimmunoassays in Table I.

Table I

| Antibody | | | | Cell Lines | | | | | | | | | | | |
| Specificity | Dilution | Expt | HLA-DR (4,w6) | Ltk⁻ | $1_4$ | $22_1$ | $22_2$ | $22_3$ | $22_4$ | $22_5$ | $22_6$ | $22_7$ | $22_8$ | $22_9$ |
|---|---|---|---|---|---|---|---|---|---|---|---|---|---|---|
| Anti HLA-A,B,C | $10^{-2}$ | 1 | 44,497 | 213 | 1,209 | 833 | | | | | | 5,999 | | 4,878 |
| "        "   " | $10^{-2}$ | 2 | 73,068 | 559 | 239 | | 572 | | | | | | | 4,405 |
| "        "   " | $10^{-2}$ | 3 | 35,679 | 1,945 | | | | 1,395 | 616 | 825 | 593 | 1,308 | | |
| Control ascite | $10^{-2}$ | 4 | 879 | 475 | 734 | | | | 1,398 | | | | 396 | 638 |
| Normal mouse serum | $10^{-2}$ | 5 | 286 | 3,264 | | 5,317 | | | | | 6,787 | 2,033 | | |
| | $10^{-3}$ | 1 | 1,306 | 29,856 | 28,192 | 45,936 | | | | | | 47,862 | | 39,934 |
| | $10^{-2}$ | 2 | 1,247 | 35,655 | 25,055 | | 49,603 | | | | | | | 43,962 |
| Anti H-2 $K^k$ | $10^{-2}$ | 3 | 3,075 | 48,115 | | | | 23,602 | 15,970 | 26,075 | 27,852 | 46,078 | | |
| | $5\times10^{-3}$ | 4 | 3,037 | 63,917 | 43,456 | | | | 54,319 | | | | 66,819 | 57,379 |
| | $10^{-2}$ | 5 | 787 | | 22,150 | 20,196 | | | | | 24,070 | 20,032 | | |
| | $10^{-2}$ | 1 | 47,389 | 431 | 391 | 5,832 | | | | | | 6,680 | | 34,646 |
| | $10^{-2}$ | 2 | 60,284 | 1,240 | 421 | | 4,292 | | | | | | | 35,517 |
| Anti HLA-DR | $10^{-3}$ | 3 | 55,875 | 1,916 | | | | 6,476 | 1,643 | 1,293 | 1,091 | 5,340 | | |
| | $10^{-2}$ | 4 | 72,822 | 3,175 | 1,303 | | | | 2,414 | | | | 5,288 | 55,328 |
| | $10^{-2}$ | 5 | 30,096 | | | 874 | 7,051 | | | | 4,935 | 6,899 | | |

† Starting material at 10 mg/ml.

As shown in Table I, all of the mouse cell lines react with H-2K$^K$ monoclonal antibodies. In addition, the human lymphoblastoid cell line (HLA-DR 4, W6), which we used as the source of our genomic DNA sequences, reacts with the anti HLA-DR monoclonal antibody mixture. Of the nine cell lines from cotransformation 22, three ($22_4$, $22_5$ and $22_6$) do not react with the anti DR monoclonals, five ($22_1$, $22_2$, $22_3$, $22_7$ and $22_8$) are DR positive, although considerably less positive than the human cell control, and one ($22_9$) reacts strongly with the antibody pool and compares favorably with the human cell control. In contrast, untransformed Ltk$^-$ cells and transformant $1_4$ (from transfection with the DR β-chain only) are negative.

We also performed some of these cell binding assays with various concentrations of the antibodies. It was evident from these assays that cotransformant $22_9$ reacts to the antibodies at the same order of magnitude as the control β-cell line.

We have also confirmed the production of these HLA-DR antigens by indirect immunofluorescence analysis. We conducted this assay by incubating $5 \times 10^5$ cells for 30 min in ice with 25 μl of $10^{-2}$ dilutions of the above-described monoclonal antibodies. After washing the cells three times in DME (10% FCS), we again incubated them for 30 min at 4°C in the presence of 5 μl rhodamine-labelled rabbit anti-mouse Ig antiserum and then washed the cells three times with PBS-BSA (10%). We then spread a drop of the resulting cell suspension with a cytofuge on microscope slides and air dried and fixed it with methanol. After mounting the slide in glycerol, we examined it with a Zeiss photomicroscope II, equipped wit an epifluorescent condensor RS III.

Because our cotransformants were propagated and analyzed as mixtures of tk$^+$ clones originating from each well, we expect that each culture contains several different clones. Accordingly, it may be that cultures showing the weaker reactivity merely have a larger percentage of DR$^-$ members than DR$^+$ members.

Finally, we analyzed the HLA-DR antigens produced at the surface of our cotransformed mouse cells by polyacrylamide gel electrophoresis. In these analyses, we labelled the cell surface proteins with $^{125}$I by iodinating $6 \times 10^6$ cells with 150 µCi $^{125}$I-iodine (3.7 GBq/ml, Amersham Int.), substantially as described by A. L. Hubbard and Z. A. Cohn, J. Cell. Biol. 55, pp. 390-405 (1972). We then incubated the freshly iodinated cells with 400 µl PBS, containing 10 µl of the mixture of HLA-DR monoclonal antibodies, as described above. After 2 h at 4°C, we washed each sample three times with cold PBS, lysed the cells with 0.4 ml buffer (150 mM NaCl, 1% deoxycholate, 1% Triton-X 100, 0.1% SDS and 10 mM Tris-HCl (pH 7.8)), sonicated them for 15 sec and centrifuged them for 15 min at 4°C in an Eppendorf centrifuge. We then immunoprecipitated the cell lysates and bound the antigen-antibody complex to protein A-sepharose, as described by E. O. Long et al., EMBO J., 1, pp. 649-54 (1982).

We eluted the labelled product from the protein A-sepharose CL-4B beads by boiling for 3 min in 10 mM Tris-HCl (pH 6.8), 15% glycerol, 2% SDS, 0.001% bromophenol-blue and an aliquot loaded onto a 12.5% polyacrylamide gel. After gel electrophoresis, we fixed the gel, dried it and exposed it to preflashed Kodak X-AR films with intensifying screens for 1 day and six days.

In this analysis, the pattern of cell surface HLA-DR α and β chains that we observed from the control β cell line [F. A. Lemonnier et al., Immunogenetics, 16, pp. 355-61 (1982)] was similar to that observed from our cotransformed cell line $22_9$. However, the mobility of the β-chain from our cotransformed cell line was slightly slower than the β-chain from the control. This slight difference could reflect a difference in the glycosylation pattern of the antigen; a difference that does not prevent recognition of the protein by anti-HLA-DR. With mouse Ltk$^-$ cells and transformed cells $1_4$, we did not observe any polypeptide chain precipitation with the HLA-DR antibody mixture.

C.   USE OF MOUSE CELLS CARRYING HUMAN
     HLA-DR ANTIGENS ON THEIR SURFACE
     TO PRODUCE MOUSE-MOUSE HYBRIDOMAS
     PRODUCING ALLOSPECIFIC HLA-DR
     MONOCLONAL ANTIBODIES

The above-described cotransformed cells that produce HLA-DR antigens at their surface may be used, as previously described, to form mouse-mouse hybridomas that produce allospecific monoclonal antibodies to those antigens. For this purpose we use our cotransformed mouse cells (e.g. $22_9$) to immunize mice by standard procedures to obtain in those mice a specific immunogenic response to the cell surface antigens of the cotransformed mouse cells. To produce hybridomas we fuse spleen cells of the immunized mice to plasmacytoma lines which are defective in the production of its own immunoglobulin chain. Following selection, we screen our mouse-mouse hybridomas for anti HLA-DR activity by large scale ELIZA or radioimmunoassay and for activity against a given DR specificity by assay with a panel of known DR positive cells.

The β-chain and In-chain DNA sequences used in the preferred embodiment of this invention were deposited in the culture collection of the American Type Culture Collection in Rockville, Maryland, on April 18, 1983:

A:   E.coli. ED 8767 (cos In-20)

B:   φ β10-charon

These deposits have been assigned accession numbers ATCC 39337 and 40067, respectively.

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than by the specific embodiments which have been presented hereinbefore by way of example.

Claims

1.    A mouse cell characterized in that it carries on its surface a selected human Class II antigen,
said cell having been cotransformed with a DNA sequence
encoding the α-chain of that selected human Class II antigen, with a DNA sequence encoding the β-chain of that
selected human Class II antigen and with a DNA sequence
encoding a selectable marker.

2.    The mouse cell according to claim 1, characterized in that said mouse cell is also cotransformed with
a DNA sequence encoding the In chain of that selected human
Class II antigen.

3.    The mouse cell according to claim 1 or 2,
characterized in that said human Class II antigen is
selected from the group consisting of HLA-DR antigens,
HLA-DC antigens, and the HLA-SB antigens.

4.    The mouse cell according to any one of the
claims 1-3, characterized in that said selectable marker
is Herpes Simplex Virus thymidine kinase (tk).

5.    A process for producing a mouse cell that
carries on its surface a selected human Class II antigen
characterized by the steps of cotransforming a mouse cell
with a DNA sequence encoding the α-chain of said selected
human Class II antigen, with a DNA sequence encoding the
β-chain of that selected human Class II antigen, and with
a DNA sequence encoding a selectable marker.

6.    The process according to claim 5, further
comprising the step of cotransforming said mouse cell with
a DNA sequence encoding the In chain of that selected human
Class II antigen.

7.    The process according to claim 5 or 6, characterized in that said human Class II antigen is selected
from the group consisting of HLA-DR antigens, HLA-DC antigens and HLA-SB antigens.

8.    A mouse-mouse hybridoma that is characterized by a mouse spleen cell selected from a mouse of the
same genetic background as a mouse cell used to immunize
that mouse, said mouse cell carrying on its surface a selected human Class II antigen and said cell having been cotransformed with a DNA sequence encoding the α-chain of a selected human Class II antigen, with a DNA sequence encoding the β-chain of that selected human Class II antigen and with a DNA sequence encoding a selectable marker.

9. The mouse-mouse hybridoma of claim 8, characterized in that said mouse cell is also transformed with a DNA sequence encoding the In chain of that selected human Class II antigen.

10. The mouse-mouse hybridoma of claim 8 or 9, characterized in that said human Class II antigen is selected from the group consisting of HLA-DR antigens, HLA-DC antigens and HLA-SB antigens.

11. A mouse-mouse hybridoma characterized by the production of a monoclonal antibody recognizing the allospecific determinants of a selected human Class II antigen.

12. A monoclonal antibody recognizing the allospecific determinants of a selected human Class II antigen.

13. The monoclonal antibody of claim 12, characterized in that such human Class II antigen is selected from the group consisting of HLA-DR antigens, HLA-DC antigens and HLA-SB antigens.

14. The use of the monoclonal antibodies of claim 12 or 13, or active fragments thereof, in typing or therapeutic processes and compositions.

15. A method for contransforming host cells comprising the steps of cotransforming the cell with at least two distinct DNA sequences, said DNA sequences encoding proteins that interact with each other after production in said host cell.

16. The method of claim 15, characterized in that said host cell is a eukaryote.

# FIGURE 1

HLA-D REGION

subregions :

GLO            SB              DR              DC              C        HLA

                                                                       B   C   A

Genes          α               α               α, α(?)
or loci :      β....?          β. β. β          β. β

Products :              Class II antigens (α/β)

2|2.

# FIGURE 2

## λ DR β-10

2 Kb